# FASCICULE DE BREVET EUROPEEN

(11) **EP 3 877 763 B1**
(45) Date de publication et mention de la délivrance du brevet: **31.08.2022**
(21) Numéro de dépôt: 19829261.7
(22) Date de dépôt: 04.11.2019
(51) Int. Cl.: G01N 33/50, A61K 8/9717, A61Q 19/08

(54) **PROCÉDÉ POUR ÉVALUER LA CAPACITÉ D'UNE SUBSTANCE OU D'UNE COMPOSITION À PRÉVENIR, RALENTIR OU ÉLIMINER LES SIGNES DU VIEILLISSEMENT DE LA PEAU OU DES LÈVRES**
VERFAHREN ZUR BEURTEILUNG DER FÄHIGKEIT EINER SUBSTANZ ODER EINER ZUSAMMENSETZUNG, UM DIE ANZEICHEN DER ALTERUNG DER HAUT ODER DER LIPPEN ZU VERHINDERN, ZU VERLANGSAMEN ODER ZU ELIMINIEREN
METHOD FOR ASSESSING THE ABILITY OF A SUBSTANCE OR OF A COMPOSITION TO PREVENT, SLOW OR ELIMINATE THE SIGNS OF AGEING OF THE SKIN OR OF THE LIPS

(30) Priorité: 06.11.2018 FR 1860191
(43) Date de publication de la demande: 15.09.2021
(73) Titulaire: Société d'Exploitation de Produits pour les Industries Chimiques SEPPIC, 75321 Paris cedex 07 (FR); BiotechMarine, 22260 Quemper Guezennec (FR)
(72) Inventeur: CATTUZZATO, Laetitia, 81100 Castres (FR); VINCENT, Gaëlle, 75321 Paris Cedex 07 (FR); LAVILLE, Rémi, 22260 Quemper Guezennec (FR); VINAT, Sandrine, 22260 Quemper Guezennec (FR); BIZE, Cécile, 75321 Paris Cedex 07 (FR)
(74) Mandataire: Air Liquide
(86) Numéro de dépôt international: PCT/FR2019/052592
(87) Numéro de publication internationale: WO 2020/094954

(56) Documents cités:
- EP-A1- 2 703 498
- WO-A1-02/39974
- WO-A1-2017/082144
- DE-A1- 10 259 966
- FR-A1- 2 971 792
- GLYN NELSON ET AL: "A senescent cell bystander effect: senescence-induced senescence : Senescence induces senescence", AGING CELL, vol. 11, no. 2, 9 février 2012 (2012-02-09), pages 345-349, XP055625700, GB ISSN: 1474-9718, DOI: 10.1111/j.1474-9726.2012.00795.x cité dans la demande
- DIMRI G P ET AL: "A BIOMARKER THAT IDENTIFIES SENESCENT HUMAN CELLS IN CULTURE AND IN AGING SKIN IN VIVO", PROCEEDINGS OF THE NATIONAL ACADEMY OF SCIENCES (PNAS), US, vol. 92, 1 septembre 1995 (1995-09-01), pages 9363-9367, XP009065201, ISSN: 0027-8424, DOI: 10.1073/PNAS.92.20.9363

## Description

La présente invention est relative à un procédé pour évaluer la capacité d'une substance chimique (S) ou d'une composition chimique (C) à prévenir ou à ralentir l'apparition des signes du vieillissement de la peau humaine ou des lèvres ou bien d'éliminer lesdits signes.

La présente invention a également pour objet une composition cosmétique ainsi qu'une formulation cosmétique.

La peau humaine constituant la première image offerte au regard d'autrui, l'amélioration de son aspect est souvent un sujet de préoccupation pour l'être humain. La peau est le reflet soit d'un état de bien-être, souvent associé à la jeunesse, soit d'un état de fatigue et/ou de vieillissement. Les consommateurs de produits cosmétiques recherchent donc des solutions pour atténuer et/ou pour prévenir les manifestations visibles liées dudit vieillissement.

Le vieillissement cutané est un phénomène qui résulte de deux processus biologiques distincts :
- le vieillissement intrinsèque dit chronologique sous dépendance des caractéristiques génétiques et donc propre à chaque individu
- le vieillissement extrinsèque influencé par des facteurs environnementaux, comme par exemple l'exposition répétée et prolongée aux rayonnements ultra-violets naturels ou artificiels (ou photo-vieillissement), à la pollution atmosphérique, à la fumée de cigarette, à divers agents oxydants; divers stress psychologiques, émotionnels et/ou nerveux.

Le vieillissement est caractérisé par des altérations, des modifications métaboliques, fonctionnelles, cellulaires, architecturales et tissulaires conduisant à des effets externes visibles. Pour le vieillissement cutané, il s'agit de l'apparition et/ou de l'accroissement de rides et ridules, d'un teint terne, d'un manque d'uniformité de ce teint (dyschromie), ou encore la modification de la texture de la peau (comme par exemple la diminution de l'épaisseur de la peau) et des propriétés notamment biomécaniques (perte d'élasticité) de la peau.

Ainsi, la peau, organe en renouvellement permanent, voit au cours du temps son renouvellement ralentir, phénomène conduisant à une accumulation de "vieilles" cellules et à une altération des mécanismes de réparation et de maintenance de celles-ci.

Les cellules de la peau entrent alors en sénescence : elles subissent un arrêt du cycle cellulaire survenant après un nombre fini de divisions, associé à de nombreux changements et à des caractéristiques phénotypiques spécifiques [1].

Les changements les plus notables sont :
- L'augmentation de la taille des cellules
- L'augmentation de la taille des noyaux des cellules
- L'augmentation de l'activité lysosomale des cellules conduisant à une augmentation de l'autophagie
- La production et la sécrétion de molécules bioactives telles que par exemple des interleukines, des facteurs de croissance, des enzymes dégradant la matrice ou des espèces réactives de l'oxygène (ou dénommées « ROS»); lesdites molécules bioactives constituant le « Senescence-Associated Secretory Phenotype (SASP) » (« phénotype sécrétoire associé à la sénescence » en français) ou « Senescence Messaging Secretome (SMS) » (« sécrétome associé à la senescence ») [2] [3]
- La modification de l'activité métabolique avec notamment une augmentation de la synthèse des métalloprotéases matricielles (MMP-1), une diminution de la synthèse de procollagène-1 et une augmentation de l'activité de la β-Galactosidase (marqueur caractéristique de la sénescence cellulaire)
- L'apparition de dommages à l'ADN
- La modification du cycle cellulaire et un ralentissement de la prolifération cellulaire.

Ce phénomène de sénescence est lié à de nombreux mécanismes dont la contagion de cellules "jeunes" par les médiateurs produits par les cellules en sénescence voisines.

Ces molécules sécrétées par ces cellules "vieilles" (SMS ou SASP) sont détectées par les cellules "jeunes" voisines, ne présentant pas les modifications cellulaires décrites ci-dessus (morphologie, métabolisme ou prolifération ...). Ces cellules voisines « jeunes », ayant été en contact avec les molécules secrétées par les cellules sénescentes (ou SMS), vont alors entrer à leur tour en sénescence et présenter les altérations morphologiques et cellulaires spécifiques du vieillissement cutané. Ce phénomène a été récemment décrit et a été appelé « vieillissement contagieux ou effet bystander » [4].

Des méthodes utilisant la co-cultures de différentes cellules de la peau, exposées aux rayonnements ultra-violets, ont été décrites pour étudier la susceptibilité des mélanocytes, des kératinocytes et des fibroblastes humains à induire un « effet bystander » ou à être sensibles à cet effet [5]. Dans ces méthodes, les différents types de cultures cellulaires ont été exposées aux rayonnements ultra-violets A, et différents marqueurs de sénescence, particulièrement les effets oxydants et les dommages à l'ADN, ont été évalués. Il ressort de cette étude que :
- la sénescence est peu induite par des expositions directes aux rayonnements ultra-violets A sur les mélanocytes, mais lesdits mélanocytes sont sensibles au « vieillissement contagieux ou effet bystander » ;
- les kératinocytes sont les cellules les plus résistantes au « vieillissement contagieux ou effet bystander » ;
- les fibroblastes ont présenté des réponses intermédiaires aussi bien à l'induction par les rayonnements ultra-violets A que pour la résistance au « vieillissement contagieux ou effet bystander ».

Cependant, cette méthode se caractérise par un temps de mise en œuvre long, et peu adaptée à une utilisation pour sélectionner des actifs anti-âge dans une démarche de criblage, très courante dans le domaine de la recherche.

Un procédé pour évaluer la capacité d'un agent biologiquement actif à prévenir ou
à ralentir l'apparition des signes du vieillissement de la peau humaine, en agissant en tant que modulateur de la sénescence cellulaire, a été décrit [6]. Ce procédé comprend une étape d'induction d'un phénotype de sénescence cellulaire par la régulation négative de l'expression du facteur de transcription FOXO3a dans des fibroblastes humains en culture.

Il apparaît que l'état de la technique ne divulgue pas de procédé in vitro de sélection d'une substance chimique ou d'une composition chimique permettant d'empêcher ou de ralentir l'apparition des signes du vieillissement de la peau humaine ou des lèvres ou bien d'éliminer lesdits signes, qui soit susceptible d'être utilisable en moyen débit dans une démarche de criblage, qui n'implique pas de cellules animales, qui soit reproductible, sensible, discriminante et efficace, et qui soit adapté au marché de la cosmétique.

Il existe donc un besoin de mettre au point un procédé de sélection d'une substance chimique ou d'une composition chimique permettant de prévenir ou de ralentir l'apparition des signes du vieillissement de la peau humaine ou des lèvres ou bien d'éliminer lesdits signes ; ledit procédé :
- n'impliquant pas la mise en œuvre de cellules animales
- soit mis en œuvre sur une durée suffisamment courte (72 heures maximum)
- ne mettant pas en œuvre de modèle d'études cliniques, ou de biopsies à partir d'études cliniques
- et par conséquent pouvant être utilisé dans une démarche de criblage, visant à tester et éventuellement sélectionner jusqu'à 12 produits réparties en quatre séries de culture.

Une solution de la présente invention est un procédé pour évaluer la capacité d'une substance chimique (S) ou d'une composition chimique (C) à prévenir ou à ralentir l'apparition des signes du vieillissement de la peau humaine ou des lèvres ou bien d'éliminer lesdits signes ; ledit procédé comprenant :
- une étape a) de mise en contact de la substance chimique ou de la composition chimique avec des cellules de fibroblastes du derme humain «jeunes» prises dans un milieu de culture au passage R6,
- un étape b) de mise en contact de cellules sénescentes de fibroblastes du derme humain avec les cellules de fibroblastes « jeunes » issues de l'étape a), et
- une étape c) de mesure de l'entrée en sénescence des cellules de fibroblastes « jeunes » et de comparaison avec une valeur de référence.

Par« substance » on entend une molécule définie par une formule, et par
« composition » un mélange de molécules.

Par« mise en contact » on entend un ajout dans le même milieu.

Par « fibroblastes du derme humain », on désigne au sens de la présente invention les cellules présentes dans le tissu conjonctif, résidentes du derme qui en assurent la cohérence et la souplesse. Il est notamment connu de l'homme du métier qu'au cours du vieillissement de la peau, le nombre de fibroblastes a tendance à diminuer, que le rapport entre les fibres de collagènes III et I évolue, et que les éléments de la matrice extracellulaire (collagène, fibres élastiques, glycosaminoglycanes) diminuent. La peau s'amincit, elle devient moins élastique, des plis ou rides apparaissent.

Par « passage Ri », on désigne au sens de la présente invention le nombre (i) de repiquage subis par les cellules, soit :
- 19 repiquages pour le passage R19
- 20 repiquages pour le passage R20
- 5 repiquages pour le passage R5
- 6 repiquages pour le passage R6.

Par« capacité à prévenir ou à ralentir l'apparition des signes du vieillissement de la peau humaine ou des lèvres ou bien d'éliminer lesdits signes » on entend au sens de la présente invention, la capacité à prolonger la durée au bout de laquelle apparaissent lesdits signes de vieillissement sur la peau humaine ou sur les lèvres.

Par "signes du vieillissement de la peau humaine ou des lèvres" on entend, au sens de l'invention, toutes modifications de l'aspect extérieur de la peau ou des lèvres dues au vieillissement, par exemple les rides et ridules, l'altération du microrelief, le manque d'élasticité et/ou de tonus de la peau, le manque de densité et/ou de fermeté de la peau humaine ou des lèvres, mais également toutes modifications internes de la peau qui ne se traduisent pas systématiquement par un aspect extérieur modifié, par exemple toutes dégradations internes de la peau consécutives à une exposition aux rayonnements ultra-violets.

Selon le cas le procédé selon l'invention peut présenter une ou plusieurs des caractéristiques ci-dessous :
- les cellules sénescentes sont des cellules de fibroblastes du derme humain pris dans un milieu de culture au passage R20.
- les cellules sénescentes sont des cellules de fibroblastes du derme humain qui ont été pris dans un milieu de culture au passage R19 avant de subir une amplification.
- les cellules de fibroblastes du derme humain « jeunes » prises dans un milieu de culture au passage R6 sont issues d'une amplification de cellules de fibroblastes du derme humain « jeunes » prises dans un milieu de culture au passage R5.
- l'étape c) de mesure de l'entrée en sénescence des cellules de fibroblastes « jeunes » comprend l'utilisation d'au moins deux marqueurs biologiques : au moins un marqueur biologique (M1) du SASP/SMS, et au moins un marqueur biologique (M2) de la sénescence.
- le marqueur biologique (M1) du SASP/SMS est choisi parmi les éléments du groupe constitué par les cytokines pro-inflammatoires, et plus particulièrement l'interleukine-1 (IL-1) extracellulaire, l'interleukine-6 (IL-6) extracellulaire, l'interleukine-8 (IL-8) extracellulaire, des facteurs de croissance, les enzymes de dégradation de la matrice telles que la MMP-1 et la MMP-3, des espèces réactives de l'oxygène ou ROS.
- le marqueur biologique (M2) de la sénescence est choisi parmi les éléments du groupe constitué par la β-galactosidase, les dommages à l'ADN persistants, et une résistance à l'apoptose.
- le marqueur biologique (M1) est l'Interleukine-6 et le marqueur biologique (M2) est l'enzyme β-galactosidase.
- à l'étape c) on compare les niveaux d'expressions des deux marqueurs biologiques (M1) et (M2) avec au moins un niveau de d'expression de référence pour chacun de ces deux marqueurs biologiques.
- les signes du vieillissement de la peau humaine ou des lèvres sont choisis parmi l'apparition des rides, des ridules ou d'une altération du microrelief; et/ou l'apparition du manque d'élasticité et/ou de tonus ; et/ou l'apparition du manque de densité et/ou de fermeté.

Par « marqueur biologique », on entend au sens de la présente demande une caractéristique qui est objectivement mesurée et évaluée comme indicateur de processus biologiques normaux, de processus pathogéniques, ou de réponse pharmacologiques à une intervention extérieure. Un marqueur biologique peut être par exemple une substance dont la détection indique un état pathologique particulier ou au contraire une substance dont la détection indique un état physiologique particulier.

Par « marqueur biologique (M1) du SASP/SMS », on désigne au sens de la présente invention un marqueur biologique tel que défini précédemment, dont la variation d'expression est corrélée avec le phénomène de vieillissement contagieux ou effet bystander, au sens d'appartenir au groupe de molécules actives contenues dans le « SASP » ou « senescent associated secreted phenotype », aussi nommé «SMS» ou « Senescence messaging secretome »)

Parmi les marqueurs biologiques (M1) du SASP/SMS dont on mesure le niveau d'expression dans l'étape e) du procédé objet de la présente invention, on peut citer les éléments du groupe de marqueurs biologiques (M1) du groupe constitué par l'interleukine-1 (IL-1) extracellulaire, l'interleukine-6 (IL-6) extracellulaire, l'interleukine-8 (IL-8), des facteurs de croissance, les enzymes de dégradation de la matrice telles que la MMP-1 et la MMP-3, des espèces réactives de l'oxygène ou ROS, et tout particulièrement l'interleukine-6 (IL-6) extracellulaire.

Au sens de la présente demande, le marqueur biologique IL-6 comprend le gène IL-6 humain (référence NCBI : Gene ID : GenBank: JQ250825.1), ainsi que les produits de ce gène. Dans un mode de réalisation particulier, le marqueur biologique IL-6 consiste en l'un des produits du gène IL-6 humain. Les produits du gène IL-6 humain comprennent le transcrit du gène IL-6 humain et la protéine humaine IL-6. Au sens de la présente demande, le « transcrit du gène IL-6 humain » est le polynucléotide dont la séquence a pour référence NCBI : GenBank: M54894.1. Par «protéine humaine IL-6», on entend au sens de la présente demande la protéine dont la séquence peptidique est la séquence de référence NCBI GenBank: AAD13886.1.

Par« marqueur biologique (M2) de la sénescence », on entend au sens de la présente demande, un marqueur biologique tel que défini précédemment, dont la variation d'expression est corrélée avec la sénescence cellulaire.

Parmi les marqueurs biologiques (M2) de la sénescence » dont on mesure le niveau d'expression dans l'étape c) du procédé objet de la présente invention, on peut citer les éléments du groupe de marqueurs biologiques (M2) constitué par l'enzyme β-galactosidase, les dommages à l'ADN persistants (associés à la détection des foyers yH2AX), une résistance à l'apoptose (associés à la détection de p53, p16, p21, IGFBP5), tout particulièrement l'enzyme β-galactosidase.

Selon un aspect particulier du procédé tel que défini précédemment, dans l'étape c), le marqueur biologique (M1) est l'Interleukine-6, le marqueur biologique (M2) est l'enzyme β-galactosidase.

Pour chacun des marqueurs biologiques (M1) et (M2), l'expression « la mesure du niveau d'expression » se réfère de manière générale soit à une mesure de la quantité de transcrits d'un gène, soit à une mesure de la quantité de molécules biologiques, et plus particulièrement des protéines et des métabolites, produites par le corps humain, soit à un niveau d'activité enzymatique.

Lorsque le niveau d'expression du marqueur biologique est mesuré au niveau nucléotidique, à savoir par la mesure de la quantité de produits du gène sous sa forme nucléotidique, toute méthode habituellement mise en œuvre par l'homme du métier pour mesurer des quantités nucléotidiques peut être mise en œuvre, et on peut ainsi citer la qRTPCR (real time quantitative polymerase chain reaction), les puces à ADN, l'hybridation in situ.

Lorsque le niveau d'expression du marqueur biologique est mesuré au niveau protéique, fonctionnel ou métabolique, à savoir en mesurant sa quantité s'il s'agit d'une protéine ou d'un métabolite, ou en mesurant sa fonction biologique, toute méthode habituellement mise en œuvre par l'homme du métier pour mesurer des quantités de protéines ou de métabolites, ou des fonctionnalités peut être mise en œuvre et on peut ainsi citer le dosage ELISA, le Western-Blot, la spectrométrie de masse, l'immunofluorescence, des techniques chromatographiques, l'activité enzymatique.

Dans le procédé objet de la présente invention, la mesure de l'expression du marqueur biologique (M1) de l'inflammation est réalisée plus particulièrement au niveau protéique.

Selon un aspect plus particulier du procédé objet de la présente invention, lorsque le marqueur biologique (M1) est l'Interleukine-6, la mesure de l'expression de l'Interleukine-6 est réalisée au niveau protéique et encore plus particulièrement avec une mesure mettant en œuvre une méthode ELISA (Enzyme-Linked ImmunoSorbent Assay) colorimétrique. Cette méthode utilise un anticorps spécifique de l'IL-6 humaine fixée au fond des puits d'une plaque de mesure. Les échantillons à doser sont ajoutés dans les puits et l'IL-6 présente dans les échantillons se fixe à l'anticorps immobilisé. Les puits sont ensuite lavés et un autre anticorps anti-IL-6 humaine biotinylé est ajouté.

Les puits sont une nouvelle fois rincés pour éliminer l'anticorps non fixé, puis de la streptavidine conjuguée à l'enzyme HRP (horseradish peroxidase) est ajoutée aux puits. Les puits sont à nouveau rincés et le substrat tetramethylbenzidine de l'HRP est ajouté aux puits.

L'intensité de la couleur se développe proportionnellement à la quantité d'IL-6 liée. La solution « stop» change la couleur du bleu au jaune, et l'intensité de couleur est mesurée à 450 nm par spectrophotométrie. La concentration en IL-6 est calculée en se référant à une gamme étalon.

La mesure de l'activité de l'enzyme β-galactosidase est réalisée au niveau métabolique, et encore plus particulièrement avec une mesure mettant en œuvre un détection enzymatique colorimétrique.

Brièvement, la β-galactosidase présent dans les cellules réagit spécifiquement avec un de ses substrats, X-gal (ou 5-bromo-4-chloro-3-indolyl-beta-D-galactopyranoside (C14H15BrClN06)) qui est déposé sur les tapis cellulaires. Ce dernier est un galactoside, un hétéroside du galactose, lié à un noyau indole substitué. Ce composé incolore est hydrolysé par la β-galactosidase, ce qui libère la partie indolique qui forme ensuite par oxydation un composé bleu, insoluble dans l'eau, qui précipite au site de la réaction. La coloration bleue est détectée/observée au microscope avec prise de photos. Pour chaque photo, le nombre de cellules marquées (cellules bleues) ainsi que le nombre de cellules totales visibles sont comptées et le nombre de cellules bleues est rapporté au nombre de cellules totales pour la normalisation.

Selon un aspect encore plus particulier du procédé objet de la présente invention, lorsque le marqueur biologique (M1) est l'Interleukine-6, la mesure de l'expression de l'Interleukine-6 est réalisée au niveau protéique, et encore plus particulièrement avec une mesure mettant en œuvre une méthode ELISA colorimétrique, et lorsque le marqueur biologique (M2) est la β-galactosidase, la mesure de l'activité de la β-galactosidase est réalisée au niveau métabolique, et encore plus particulièrement avec une mesure mettant en œuvre une détection enzymatique colorimétrique.

Par un « niveau d'expression de référence » d'un marqueur biologique, on désigne tout niveau d'expression dudit marqueur biologique utilisé à titre de référence.

Dans le cadre de la présente invention, un niveau d'expression de référence du marqueur biologique (M1) du SASP/SMS peut être obtenu en mesurant le niveau d'expression dudit marqueur biologique (M1) :
- Dans le milieu de culture cellulaire des cellules de fibroblastes du derme humain « jeunes » au passage R6 (cellules « jeunes » au passage R6 non mises en contact avec des cellules sénescentes et non traitées avec la substance ou la composition chimique). Cette mesure du niveau d'expression du marqueur (M1) sur les cellules non traitées et non stressées est notée N1, et/ou
- Dans un milieu de culture cellulaire obtenu par mise en contact des cellules de fibroblastes du derme humain « jeunes » prises au passage R6 avec des cellules de fibroblastes du derme humain sénescentes prises au passage R20; cette mesure du niveau d'expression du marqueur (M1) sur les cellules non traitées mais stressées est notée N1₀ ; et/ou
- Dans le milieu de culture cellulaire obtenu à l'issue de la mise en œuvre de l'étape b) du procédé objet de la présente invention, lorsque la substance (S) mise en œuvre dans l'étape a) du procédé objet de la présente invention est une substance choisie parmi les éléments du groupe constitué par la vitamine E, la vitamine C. Cette mesure du niveau d'expression du marqueur biologique (M1) pour une substance (S) de référence est notée N1Ref.

Dans la cadre de la présente invention, un niveau d'expression de référence du marqueur biologique (M2) de la sénescence peut être obtenu en mesurant le niveau d'expression dudit marqueur biologique (M2) :
- Dans le milieu de culture cellulaire des cellules de fibroblastes du derme humain « jeunes » au passage R6 (cellules « jeunes » au passage R6 non mises en contact avec des cellules sénescentes et non traitées avec la substance ou la composition chimique) ; cette mesure du niveau d'expression du marqueur biologique (M2) sur les cellules non traitées et non stressées est notée N2, et/ou
- Dans un milieu de culture cellulaire obtenu par mise en contact des cellules de fibroblastes du derme humain « jeunes » prises au passage R6 avec des cellules de fibroblastes du derme humain sénescentes prises au passage R20 ; cette mesure du niveau d'expression du marqueur biologique (M2) sur les cellules non traitées mais stressées est notée N20, et/ou
- Dans le milieu de culture cellulaire obtenu à l'issue de la mise en œuvre de l'étape b) du procédé objet de la présente invention, lorsque la substance (S) mise en œuvre dans l'étape a) du procédé objet de la présente invention est une substance choisie parmi les éléments du groupe constitué par la vitamine E, la vitamine C. Cette mesure du niveau d'expression du marqueur biologique (M2) pour une substance (S) de référence est notée N2Ref.

Grâce au procédé tel que défini précédemment, il est possible de sélectionner ladite substance (S) ou de ladite composition (C) comme ingrédient destiné à prévenir ou à ralentir l'apparition des signes du vieillissement de la peau humaine ou des lèvres ou bien d'éliminer lesdits signes, et apte à être utilisée en tant que telle, ou pour préparer des compositions à usage topique à destination des industries de la cosmétique ou de la dermocosmétique ou de la pharmacie.

Grâce au procédé tel que défini précédemment, une substance (S) ou une composition (C) sera sélectionnée pour être utilisée en tant que telle, ou pour préparer des compositions à usage topique la comprenant, pour prévenir ou ralentir l'apparition des signes du vieillissement de la peau humaine ou des lèvres ou bien d'éliminer lesdits signes, si :
- Le niveau d'expression mesuré pour ladite substance (S) ou pour ladite composition (C), noté N1i, est inférieur au niveau d'expression de référence du marqueur biologique (M1) du SASP/SMS N10, et si
- Le niveau d'expression mesuré pour ladite substance (S) ou pour ladite composition (C), noté N2i, est inférieur au niveau d'expression de référence du marqueur biologique (M2) N20 de la sénescence.

Une substance (S) ou une composition (C) sera plus particulièrement sélectionnée pour être utilisée en tant que telle, ou pour préparer des compositions cosmétique à usage topique la comprenant, pour prévenir ou ralentir l'apparition des signes du vieillissement de la peau humaine ou des lèvres ou bien d'éliminer lesdits signes, si :
- Le rapport R1= [(N10 - N1i) x 100] / [(N10 - N1)] est supérieur ou égal à n1; et si
- Le rapport R2 = [(N20 - N2i) x 100] / [(N20 - N2] est supérieur ou égal à n2, avec ni supérieur ou égal à 15, et plus particulièrement supérieur ou égal à 30, n2 supérieur ou égal à 60, et plus particulièrement supérieur ou égal à 70, N1, N10, N2 et N20 étant tels que définis précédemment ou
ni tel que défini ci-dessus est plus particulièrement supérieur ou égal à 15, tout particulièrement supérieur ou égal à 30, et encore plus particulièrement supérieur ou égal à 40; et n2 tel que défini ci-dessus est plus particulièrement supérieur ou égal à 60, tout particulièrement supérieur ou égal à 70, et encore plus particulièrement supérieur ou égal à 80.

Grâce au procédé tel que défini précédemment, une substance (S) ou une composition (C) sera plus particulièrement sélectionnée si ni est supérieur ou égal à 30 et si n2 est supérieur ou égal à 70.

Une composition cosmétique (CA) est également décrite. Elle comprend pour 100% de sa masse, de 0,1% à 25 % massique d'au moins un composé de formule générale (I) : 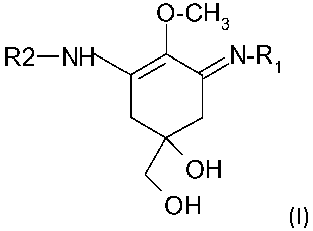 dans laquelle :
- R₁ représente le radical de formule (Ia)

   -CH₂-CO₂H (Ia)

   , et R₂ représente l'atome d'hydrogène, ou bien
- R₁ représente le radical de formule (Ib)

   -CH(CO₂H)-CH₂-CH₂-OH (Ib)

   , et R₂ représente l'atome d'hydrogène, ou bien
- R₁ représente le radical de formule (la) et R₂ représente le radical hydroxyéthyl (Ic) :

   HO-CH₂-CH₂- (Ic)

   , ou bien
- R₁ représente le radical de formule (la) et R₂ représente le radical hydroxyméthyléthyl (Id) :

   HO-C(CH₃)(CH₂- CH₃) (Id)

   , ou bien
- R₁ représente le radical de formule (la) et R₂ représente le radical hydroxypropyl (Ie) :

   HO-CH₂-CH₂-CH₂- (Ie)

   , ou bien
- R₁ représente le radical de formule (la) et R₂ représente le radical propèn-(E)yl (If), ou bien
- R₁ représente le radical de formule (la) et R₂ représente le radical propèn-(Z)yl (Ig), ou bien
- R₁ et R₂ représentent tous les deux le radical de formule (la), ou bien
- R₁ représente le radical de formule (Ii)

   -CH(CO₂H)-CH(OH)-CH₃ (Ii)

   et R₂ représente l'atome d'hydrogène, ou bien
- R₁ représente le radical de formule (Ii) et R₂ représente le radical méthyl, ou bien
- R₁ représente le radical de formule (Ib) et R₂ représente le radical méthyl, ou bien
- R₁ représente le radical de formule (la) et R₂ représente le radical de formule (Ij)

   -CH(CO₂H)-CH₃ (Ij)

   , ou bien
- R₁ représente le radical de formule (la) et R₂ représente le radical de formule (Ib), ou bien
- R₁ représente le radical de formule (la) et R₂ représente le radical de formule (Ik)

   -CH(CO₂H)-CH(CH₃)₂ (Ik)

   , ou bien
- R₁ représente le radical de formule (la) et R₂ représente le radical de formule (Ii), ou bien
- R₁ représente le radical de formule (la) et R₂ représente le radical de formule (II)

   --CH(CO₂H)-CH₂(CO₂H) (II)

   , ou bien
- R₁ représente le radical de formule (la) et R₂ représente le radical de formule (Im)

   --CH₂-CH=CH-CO₂H (Im)

La composition cosmétique (CA) comprend pour 100% de sa masse, de 0,1% à 25 % massique d'au moins un composé de formule générale (I) dans laquelle :
- R₁ et R₂ représentent tous les deux le radical de formule (la), ou bien
- R₁ représente le radical de formule (la) et R₂ représente l'atome d'hydrogène, ou bien
- R₁ représente le radical de formule (la) et R₂ représente le radical (Ic), ou bien
- R₁ représente le radical de formule (la) et R₂ représente le radical de formule (Ib), ou bien
- R₁ représente le radical de formule (la) et R₂ représente le radical (If).

La composition cosmétique (CA) pourrait être utilisée pour empêcher ou ralentir l'apparition des signes du vieillissement de la peau humaine ou des lèvres ou bien éliminer lesdits signes.

La composition cosmétique (CA) pourrait être préparée par mélange de ses composants dans les proportions massiques souhaitées, à température ambiante ou supérieure au point de fusion des ingrédients si nécessaire, et sous agitation mécanique modérée (50 à 150 tours.min⁻¹).

La composition cosmétique (CA) pourrait aussi être préparée par toute technique d'extraction connu de l'homme du métier, et appliquée à des algues rouges, et plus particulièrement à des algues de la division des Rhodophyta, de la classe des Florideophycae, de l'ordre des Acrochaetiales, Acrosymphytales, Ahnfeltiales, Balbianiales, Balliales, Batrachospermales, Bonnemaisoniales, Ceramiales, Colaconematales, Corallinales, Entwisleiales, Florideophyceae incertae sedis, Gelidiales, Gigartinales, Gracilariales, Halymeniales, Hildenbrandiales, Nemaliales, Nemastomatales, Palmariales, Peyssonneliales, Pihiellales, Plocamiales, Rhodachlyales, Rhodogorgonales, Rhodymeniales, Sebdeniales, Sporolithales et Thoreales.

La composition cosmétique (CA) telle que définie ci-dessus peut aussi être préparée par toute technique d'extraction connu de l'homme du métier, et appliquée à des algues rouges de l'ordre des Bonnemaisoniales, de la famille des Bonnemaisoniaceae, des genres/espèces Asparagopsis armata et Asparagopsis taxiformis.

Une formulation cosmétique à usage topique comprenant au moins un excipient cosmétiquement acceptable et une quantité efficace de la composition cosmétique (CA) telle que définie précédemment est décrite.

La formulation cosmétiqu sera une formulation pour empêcher ou ralentir l'apparition des signes du vieillissement de la peau humaine ou des lèvres ou bien éliminer lesdits signes.

Ladite formulation cosmétique à usage topique sera généralement étalée sur la surface de la peau à traiter, puis la peau est massée quelques instants.

L'expression "à usage topique" utilisée dans la définition de la formulation cosmétique, signifie que ladite formulation est mise en œuvre par application sur la peau, qu'il s'agisse d'une application directe dans le cas d'une formulation cosmétique ou d'une application indirecte lorsque la formulation cosmétique est imprégnée sur un support destiné à être mis en contact avec la peau (papier, lingette, textile, dispositif transdermique, etc...).

L'expression « cosmétiquement acceptable » utilisée dans la définition de la formulation cosmétique à usage topique, signifie selon la directive du Conseil de la Communauté Economique Européenne N°76/768/CEE du 27 juillet 1976 modifiée par la directive N°93/35/CEE du 14 juin 1993, que ladite formulation à usage topique comprend toute substance ou préparation destinée à être mise en contact avec les diverses parties du corps humain (épiderme, système pileux et capillaire, ongles, lèvres et organes génitaux) ou avec les dents et les muqueuses buccales en vue, exclusivement et principalement, de les nettoyer, de les parfumer, d'en modifier l'aspect et/ou d'en corriger les odeurs corporelles et/ou de les protéger ou de les maintenir en bon état.

Par quantité efficace de la composition (A) telle que définie précédemment et présente dans la formulation cosmétique à usage topique , on entend pour 100% de la masse de ladite formulation cosmétique à usage topique, la quantité comprise entre 0,1% et 5% massique, plus particulièrement entre 0,1% et 3% massique, et encore plus particulièrement entre 0,5% et 2,5 % massique de composition (A).

Les formulations cosmétiques à usage topique se présentent généralement sous forme de solutions aqueuses ou hydro-alcooliques ou hydro-glycoliques, sous forme d'une suspension, d'une émulsion, d'une microémulsion ou d'une nanoémulsion, qu'elles soient de type eau-dans-huile, huile-dans-eau, eau-dans-huile-dans-eau ou huile-dans-eau-dans-huile, ou sous forme d'une poudre.

Les formulations cosmétiques à usage topique peuvent être conditionnées dans un flacon, dans un dispositif de type "flacon" pompe, sous forme pressurisées dans un dispositif aérosol, dans un dispositif muni d'une paroi ajourée comme une grille ou dans un dispositif muni d'un applicateur à billes (dit "roll-on").

De façon générale, la composition (CA), présente dans les formulations cosmétiques à usage topique est associé à des additifs chimiques habituellement mis en œuvre dans le domaine des formulations à usage topique, comme les tensioactifs moussants et/ou détergents, les tensioactifs épaississants et/ou gélifiants, les agents épaississants et/ou gélifiants, les agents stabilisants, les composés filmogènes, les solvants et co-solvants, les agents hydrotropes, les eaux thermales ou minérales les agents plastifiants, les agents émulsionnants et co-émulsionnants, les agents opacificants, les agents nacrants, les agents surgraissants, les séquestrants, les agents chélatants, les huiles, les cires, les agents antioxydants, les parfums, les huiles essentielles, les agents conservateurs, les agents conditionneurs, les agents déodorants, les agents blanchissants destinés à la décoloration des poils et de la peau, les principes actifs destinés à apporter une action traitante et/ou protectrice vis à vis de la peau ou des cheveux, les filtres solaires, les charges minérales ou les pigments, les particules procurant un effet visuel ou destinées à l'encapsulation d'actifs, les particules exfoliantes, les agents de texture, les azurants optiques, les répulsifs pour les insectes.

Comme exemples de tensioactifs moussants et/ou détergents que l'on peut associer à la composition (CA) dans les formulations cosmétiques à usage topique , on peut citer les tensioactifs moussants et/ou détergents anioniques, cationiques, amphotères ou non ioniques.

Parmi les tensioactifs anioniques moussants et/ou détergents, on peut citer les sels de métaux alcalins, de métaux alcalino-terreux, d'ammonium, d'amines, ou d'aminoalcools d'alkylether sulfates, d'alkyl sulfates, d'alkylamidoéther sulfates, d'alkylaryl polyéthersulfates, de monoglycérides sulfates, d'alpha-oléfinesulfonates, de paraffines sulfonates, d'alkyl phosphates, d'alkyléther phosphates, d'alkyl sulfonates, d'alkylamide sulfonates, d'alkylaryl sulfonates, d'alkyl carboxylates, d'alkyl sulfosuccinates, d'alkyléther sulfosuccinates, d'alkylamide sulfosuccinates, d'alkyl sulfoacétates, d'alkyl sarcosinates, d'acyl iséthionates, de N-acyl taurates, d'acyl lactylates, de dérivés N-acylés d'acides aminés, de dérivés N-acylés de peptides, de dérivés N-acylés de protéines, de dérivés N-acylés d'acides gras.

Parmi les tensioactifs amphotères moussants et/ou détergents, on peut citer les alkylbétaïnes, les alkylamidobétaïnes, les sultaïnes, les alkylamidoalkylsulfobétaïnes, les dérivés d'imidazolines, les phosphobétaïnes, les amphopolyacétates et les amphopropionates.

Parmi les tensioactifs cationiques moussants et/ou détergents, on peut citer particulièrement les dérivés d'ammoniums quaternaires.

Parmi les tensioactifs non ioniques moussants et/ou détergents, on peut citer plus particulièrement les alkylpolyglycosides comportant un radical aliphatique, linéaire ou ramifié, saturé ou insaturé, et comportant de 8 à 16 atomes de carbone, comme l'octyl polyglucoside, le décyl polyglucoside, l'undécylényl polyglucoside, le dodécyl polyglucoside, le tétradécyl polyglucoside, l'hexadécyl polyglucoside, le 1-12 dodécanediyl polyglucoside ; les dérivés d'huile de ricin hydrogénée éthoxylés comme le produit commercialisé sous le nom INCI « Peg-40 hydrogenated castor oil » ; les polysorbates comme le Polysorbate 20, le Polysorbate 40, le Polysorbate 60, le Polysorbate 70, le Polysorbate 80, le Polysorbate 85 ; les amides de coprah ; les N-alkylamines.

Comme exemples de tensioactifs épaississants et/ou gélifiants que l'on peut associer à la composition (CA) dans les formulations cosmétiques à usage topique , on peut citer les esters gras d'alkylpolyglycosides éventuellement alcoxylés, comme les esters de méthylpolyglucoside éthoxylés tels que le PEG 120 méthyl glucose trioléate et le PEG 120 méthyl glucose dioléate commercialisés respectivement sous les appellations GLUCAMATE^{™} LT et GLUMATE^{™} DOE120; les esters gras alcoxylés tels que le PEG 150 pentaérythrytyl tétrastéarate commercialisé sous l'appellation CROTHIX^{™} DS53, le PEG 55 propylène glycol oléate commercialisé sous l'appellation ANTIL^{™} 141; les carbamates de polyalkylène glycols à chaînes grasses comme le PPG-14 laureth isophoryl dicarbamate commercialisé sous l'appellation ELFACOS^{™} T211, le PPG-14 palmeth-60 hexyl dicarbamate commercialisé sous l'appellation ELFACOS^{™} GT2125.

Comme exemples d'agents épaississants et/ou gélifiants que l'on peut associer à la composition (CA) dans les formulations cosmétiques à usage topique , on peut citer les polymères de type polyélectrolytes, linéaires ou branchés ou réticulés, comme l'homopolymère de l'acide acrylique partiellement ou totalement salifié, l'homopolymère de l'acide méthacrylique partiellement ou totalement salifié, l'homopolymère de l'acide-2-méthyl-[(1-oxo-2-propényl)amino]-1-propanesulfonique (AMPS) partiellement ou totalement salifié, les copolymères de l'acide acrylique et de l'AMPS, les copolymères de l'acrylamide et de l'AMPS, les copolymères de la vinylpyrolidone et de l'AMPS, les copolymères de l'AMPS et de l'acrylate de (2-hydroxyéthyle), les copolymères de l'AMPS et du méthacrylate de (2-hydroxyéthyle), les copolymères de l'AMPS et de l'hydroxyéthylacrylamide, les copolymères de l'AMPS et du N,N-diméthyl acrylamide, les copolymères de l'AMPS et du tris(hydroxy- methyl)acrylamido methane (THAM), les copolymères de l'acide acrylique ou méthacrylique et de l'acrylate de (2-hydroxy éthyle), les copolymères de l'acide acrylique ou méthacrylique et du méthacrylate de (2-hydroxy éthyle), les copolymères de l'acide acrylique ou méthacrylique et de l'hydroxyéthylacrylamide, les copolymères de l'acide acrylique ou méthacrylique et du THAM, les copolymères de l'acide acrylique ou méthacrylique et du N,N-diméthyl acrylamide, les terpolymères de l'acide acrylique ou méthacrylique, de l'AMPS et de l'acrylate de (2-hydroxy éthyle), les terpolymères de l'acide acrylique ou méthacrylique, de l'AMPS et du méthacrylate de (2-hydroxy éthyle), les terpolymères de l'acide acrylique ou méthacrylique, de l'AMPS et du THAM, les terpolymères de l'acide acrylique ou méthacrylique, de l'AMPS et du N,N-diméthyl acrylamide, les terpolymères de l'acide acrylique ou méthacrylique, de l'AMPS et de l'acrylamide, les copolymères de l'acide acrylique ou de l'acide méthacrylique et d'acrylates d'alkyle dont la chaîne carbonée comprend entre quatre et trente atomes de carbone et plus particulièrement entre dix et trente atomes de carbone, les copolymères de l'AMPS et d'acrylates d'alkyle dont la chaîne carbonée comprend entre quatre et trente atomes de carbone et plus particulièrement entre dix et trente atomes de carbone, les terpolymère linéaire, branché ou réticulé d'au moins un monomère possédant une fonction acide fort, libre, partiellement salifiée ou totalement salifiée, avec au moins un monomère neutre, et au moins un monomère de formule (VIII) :

CH₂=C(R'₃)-C(=O)-[CH₂-CH₂-O]ₙ-R'₄ (VIII)

dans laquelle R'3 représente un atome d'hydrogène ou un radical méthyle, R'4 représente un radical alkyle linéaire ou ramifié comportant de huit à trente atomes de carbone et n représente un nombre supérieur ou égal à un et inférieur ou égal à cinquante.

Les polymères de type polyélectrolytes, linéaires ou branchés ou réticulés que l'on peut associer à la composition (CA) dans les formulations cosmétiques à usage topique peuvent se présenter sous la forme d'une solution, d'une suspension aqueuse, d'une émulsion eau-dans-huile, d'une émulsion huile-dans-eau, d'une poudre. Les polymères de type polyélectrolytes, linéaires ou branchés ou réticulés que l'on peut associer au composé de formule (I) ou à la composition (C1) dans les formulations cosmétiques à usage topique peuvent être sélectionnés parmi les produits commercialisés sous les appellations SIMULGEL^{™} EG, SIMULGEL^{™}EPG, SEPIGEL^{™} 305, SIMULGEL^{™} 600, SIMULGEL^{™} NS, SIMULGEL^{™} INS 100, SIMULGEL^{™} FL, SIMULGEL^{™} A, SIMULGEL^{™} SMS 88, SEPINOV^{™}EMT 10, SEPIPLUS^{™}400, SEPIPLUS^{™}265, SEPIPLUS^{™}S, SEPIMAX^{™}Zen, ARISTOFLEX^{™}AVC, ARISTOFLEX^{™}AVS, NOVEMER^{™}EC-1, NOVEMER^{™}EC 2, ARISTOFLEX^{™}HMB, COSMEDIA^{™}SP, FLOCARE^{™}ET 25, FLOCARE^{™}ET 75, FLOCARE^{™}ET 26, FLOCARE^{™}ET 30, FLOCARE^{™}ET 58, FLOCARE^{™}PSD 30, VISCOLAM^{™}AT 64, VISCOLAM^{™}AT 100.

Comme exemples d'agents épaississants et/ou gélifiants que l'on peut associer à la composition (CA) dans les formulations cosmétiques à usage topique , on peut citer les polysaccharides constitués uniquement d'oses, comme les glucanes ou homopolymères du glucose, les glucomannoglucanes, les xyloglycanes, les galactomannanes dont le degré de substitution (DS) des unités de D-galactose sur la chaîne principale de D-mannose est compris entre 0 et 1, et plus particulièrement entre 1 et 0,25, comme les galactomannanes provenant de la gomme de cassia (DS = 1/5), de la gomme de caroube (DS = 1/4), de la gomme de tara (DS = 1/3), de la gomme de guar (DS = 1/2), de la gomme de fenugrec (DS = 1 ).

Comme exemples d'agents épaississants et/ou gélifiants que l'on peut associer à la composition (CA) dans les formulations cosmétiques à usage topique, on peut citer les polysaccharides constitués de dérivés d'oses, comme les galactanes sulfatés et plus particulièrement les carraghénanes et l'agar, les uronanes et plus particulièrement les algines, les alginates et les pectines, les hétéropolymères d'oses et d'acides uroniques et plus particulièrement la gomme xanthane, la gomme gellane, les exsudats de gomme de arabique et de gomme de karaya, les glucosaminoglycanes.

Comme exemples d'agents épaississants et/ou gélifiants que l'on peut associer à la composition (CA) dans les formulations cosmétiques à usage topique on peut citer la cellulose, les dérivés de cellulose comme la méthyl-cellulose, l'éthyl-cellulose, l'hydroxypropyl cellulose, les silicates, l'amidon, les dérivés hydrophiles de l'amidon, les polyuréthanes.

Comme exemples d'agents stabilisants que l'on peut associer à la composition (CA) dans les formulations cosmétiques à usage topique , on peut citer les cires microcristallines, et plus particulièrement l'ozokérite, les sels minéraux tels que le chlorure de sodium ou le chlorure de magnésium, les polymères siliconés tels que les copolymères polysiloxane polyalkyl polyéther.

Comme exemples de solvants que l'on peut associer à la composition (CA) dans les formulations cosmétiques à usage topique , on peut citer l'eau, les solvants organiques comme le glycérol, le diglycérol, les oligomères du glycérol, l'éthylène glycol, le propylène glycol, le butylène glycol, le 1,3-propanediol, le 1,2-propanediol, l'hexylèneglycol, le diéthylèneglycol, le xylitol, l'érythritol, le sorbitol, les alcools hydrosolubles tels que l'éthanol, l'isopropanol ou le butanol, les mélanges d'eau et desdits solvants organiques.

Comme exemples d'eaux thermales ou minérales que l'on peut associer à la composition (CA) dans les formulations cosmétiques à usage topique , on peut citer les eaux thermales ou minérales ayant une minéralisation d'au moins 300 mg/l, en particulier l'eau d'Avene, l'eau de Vittel, les eaux du bassin de Vichy, l'eau d'Uriage, l'eau de la Roche Posay, l'eau de la Bourboule, l'eau d'Enghien-les-bains, l'eau de Saint-Gervais-les bains, l'eau de Néris-les-bains, l'eau d'Allevard-les-bains, l'eau de Digne, l'eau des Maizieres, l'eau de Neyrac-les-bains, l'eau de Lons le Saunier, l'eau de Rochefort, l'eau de Saint Christau, l'eau des Fumades et l'eau de Tercis-les-bains.

Comme exemples d'agents hydrotropes que l'on peut associer à la composition (CA) dans les formulations cosmétiques à usage topique , on peut citer les xylènes sulfonates, les cumènes sulfonates, l'hexylpolyglucoside, le 2-éthylhexylpolyglucoside, le n-heptylpolyglucoside.

Comme exemples d'agents tensioactifs émulsionnants que l'on peut associer à la composition (CA) dans les formulations cosmétiques à usage topique , on peut citer les tensioactifs non ioniques, des tensioactifs anioniques, des tensioactifs cationiques.

Comme exemples de tensioactifs non-ioniques émulsionnants que l'on peut associer à la composition (CA) dans les formulations cosmétiques à usage topique , on peut citer les esters d'acides gras et de sorbitol, comme les produits commercialisés sous les appellations MONTANE^{™}40, MONTANE^{™}60, MONTANE^{™}70, MONTANE^{™}80 et MONTANE^{™}85 ; les compositions comprenant du stéarate de glycérol et l'acide stéarique éthoxylé entre 5 moles et 150 moles d'oxyde d'éthylène, comme la composition comprenant de l'acide stéarique éthoxylé à 135 moles d'oxyde d'éthylène et du stéarate de glycérol commercialisée sous l'appellation SIMULSOL^{™} 165 ; les esters de mannitan; les esters de mannitan éthoxylés ; les esters de sucrose ; les esters de méthylglucoside ; les alkylpolyglycosides comportant un radical aliphatique, linéaire ou ramifié, saturé ou insaturé, et comportant de 14 à 36 atomes de carbone, comme le tétradécyl polyglucoside, l'hexadécyl polyglucoside, l'octadécyl polyglucoside, l'hexadécyl polyxyloside, l'octadécyl polyxyloside, l'eicosyl polyglucoside, le dodécosyl polyglucoside, le 2-octyldodécyl polyxyloside, le 12-hydroxystéaryl polyglucoside ; les compositions d'alcools gras linéaires ou ramifiés, saturés ou insaturés, et comportant de 14 à 36 atomes de carbone, et d' alkylpolyglycosides tels que décrits précédemment, par exemple les compositions commercialisées sous les noms de marque MONTANOV^{™}68, MONTANOV^{™}14, MONTANOV^{™}82, MONTANOV^{™}202, MONTANOV^{™}S, MONTANOV^{™}WO18, MONTANOV^{™}L, FLUIDANOV^{™}2OX et EASYNOV^{™}.

Comme exemples de tensioactifs anioniques que l'on peut associer à la composition (CA) dans les formulations cosmétiques à usage topique , on peut citer le glycéryl stéarate citrate, le cétéarylsulfate, les savons comme le stéarate de sodium ou le stéarate de triéthanolammonium, les dérivés N-acylés d'acides aminés salifiés comme par exemple le stéaroyl glutamate.

Comme exemples de tensioactifs cationiques émulsionnants que l'on peut associer à la composition (CA) dans les formulations cosmétiques à usage topique , on peut citer les aminoxydes, le quaternium-82 et les tensioactifs décrits dans la demande de brevet WO96/00719 et principalement ceux dont la chaîne grasse comprend au moins 16 atomes de carbone.

Comme exemples d'agents opacifiants et/ou nacrants que l'on peut associer à la composition (CA) dans les formulations cosmétiques à usage topique , on peut citer le palmitate de sodium, le stéarate de sodium, l'hydroxystéarate de sodium, le palmitate de magnésium, le stéarate de magnésium, l'hydroxystéarate de magnésium, le monostéarate d'éthylène glycol, le distéarate d'éthylène glycol, le monostéarate de polyéthylène glycol, le distéarate de polyéthylène glycol, les alcools gras comportant de 12 à 22 atomes de carbone.

Comme exemples d'agents de texture que l'on peut associer à la composition (CA) dans les formulations cosmétiques à usage topique on peut citer des dérivés N-acylés d'acides aminés, comme la lauroyl lysine commercialisée sous l'appellation AMINOHOPE^{™}LL, l'octenyl starch succinate commercialisé sous l'appellation DRYFLO^{™}, le myristyl polyglucoside commercialisé sous l'appellation MONTANOV^{™} 14, les fibres de cellulose, les fibres de coton, les fibres de chitosane, le talc, la séricite, le mica.

Comme exemples d'agents déodorants que l'on peut associer à la composition (CA) dans les formulations cosmétiques à usage topique , on peut citer les silicates alcalins, les sels de zinc comme le sulfate de zinc, le gluconate de zinc, le chlorure de zinc, le lactate de zinc; les sels d'ammonium quaternaires comme les sels de cétyltriméthylammonium, les sels de cétylpyridinium ; les dérivés du glycérol comme le caprate de glycérol, le caprylate de glycérol, le caprate de polyglycérol ; le 1,2 décanediol ; le 1,3 propanediol; l'acide salicylique; le bicarbonate de sodium; les cyclodextrines ; les zéolithes métalliques; le TRICLOSAN^{™} ; le bromohydrate d'aluminium, les chlorhydrates d'aluminium, le chlorure d'aluminium, le sulfate d'aluminium, les chlorhydrates d'aluminium et de zirconium, le trichlorhydrate d'aluminium et de zirconium, le tétrachlorhydrate d'aluminium et de zirconium, le pentachlorhydrate d'aluminium et de zirconium, l'octochlorhydrate d'aluminium et de zirconium, le sulfate d'aluminium, le lactate de sodium et d'aluminium, les complexes de chlorhydrate d'aluminium et de glycol, comme le complexe de chlorhydrate d'aluminium et de propylène glycol, le complexe de dichlorhydrate d'aluminium et de propylène glycol, le complexe de sesquichlorhydrate d'aluminium et de propylène glycol, le complexe de chlorhydrate d'aluminium et de polyéthylène glycol, le complexe de dichlorhydrate d'aluminium et de polyéthylène glycol, le complexe de sesquichlorhydrate d'aluminium et de polyéthylène glycol.

Comme exemples d'huiles que l'on peut associer à la composition (CA) dans les formulations cosmétiques à usage topique , on peut citer les huiles minérales telles que l'huile de paraffine, l'huile de vaseline, les isoparaffines ou les huiles blanches minérales ; les huiles d'origine animale, telles que le squalène ou le squalane ;les huiles végétales, telles que le phytosqualane, l'huile d'amandes douces, l'huile de coprah, l'huile de ricin, l'huile de jojoba, l'huile d'olive, l'huile de colza, l'huile d'arachide, l'huile de tournesol, l'huile de germes de blé, l'huile de germes de maïs, l'huile de soja, l'huile de coton, l'huile de luzerne, l'huile de pavot, l'huile de potiron, l'huile d'onagre, l'huile de millet, l'huile d'orge, l'huile de seigle, l'huile de carthame, l'huile de bancoulier, l'huile de passiflore, l'huile de noisette, l'huile de palme, le beurre de karité, l'huile de noyau d'abricot, l'huile de calophyllum, l'huile de sysymbrium, l'huile d'avocat, l'huile de calendula, les huiles issues de fleurs ou de légumes les huiles végétales éthoxylées ; les huiles synthétiques comme les esters d'acides gras tels que le myristate de butyle, le myristate de propyle, le myristate d'isopropyle, le myristate de cétyle, le palmitate d'isopropyle, le palmitate d'octyle, le stéarate de butyle, le stéarate d'hexadécyle, le stéarate d'isopropyle, le stéarate d'octyle, le stéarate d'isocétyle, l'oléate dodécyle, le laurate d'hexyle, le dicaprylate de propylèneglycol, les esters dérivés d'acide lanolique, tels que le lanolate d'isopropyle, le lanolate d'isocétyle, les monoglycérides, diglycérides et triglycérides d'acides gras comme le triheptanoate de glycérol, les alkylbenzoates, les huiles hydrogénées, les poly(alpha-oléfine), les polyoléfines comme le poly(isobutane), les isoalcanes de synthèse comme l'isohexadécane, l'isododécane, les huiles perfluorées; les huiles de silicone comme les diméthylpolysiloxanes, les méthylphényl - polysiloxanes, les silicones modifiées par des amines, les silicones modifiés par des acides gras, les silicones modifiés par des alcools, les silicones modifiés par des alcools et des acides gras, des silicones modifiés par des groupements polyéther, des silicones époxy modifiés, des silicones modifiées par des groupements fluorés, des silicones cycliques et des silicones modifiées par des groupements alkyles. Par « huiles », on entend dans la présente demande les composés et/ou les mélanges de composés insolubles dans l'eau, se présentant sous un aspect liquide à une température de 25°C.

Comme exemples de cires que l'on peut associer à la composition (CA) dans les formulations cosmétiques à usage topique , on peut citer la cire d'abeille, la cire de carnauba, la cire de candelilla, la cire d'ouricoury, la cire du Japon, la cire de fibre de liège, la cire de canne à sucre, les cires de paraffines, les cires de lignite, les cires microcristallines, la cire de lanoline ; l'ozokérite ; la cire de polyéthylène ; les cires de silicone ; les cires végétales ; les alcools gras et les acides gras solides à température ambiante ; les glycérides solides à température ambiante. Par « cires », on entend dans la présente demande les composés et/ou les mélanges de composés insolubles dans l'eau, se présentant sous un aspect solide à une température supérieure ou égale à 45°C.

Comme exemples de principes actifs que l'on peut associer à la composition (CA) dans les formulations cosmétiques à usage topique , on peut citer les vitamines et leurs dérivés, notamment leurs esters, tels que le rétinol (vitamine A) et ses esters (palmitate de rétinyle par exemple), l'acide ascorbique (vitamine C) et ses esters, les dérives de sucre de l'acide ascorbique (comme l'ascorbyl glucoside), le tocophérol (vitamine E) et ses esters (comme l'acétate de tocophérol), les vitamines B3 ou B10 (niacinamide et ses dérivés); les composés montrant une action éclaircissante ou dépigmentante de la peau comme le ω-undecelynoyl phénylalanine commercialisé sous l'appellation SEPIWHITE^{™}MSH, le SEPICALM^{™}VG, le mono ester et/ou le diester de glycérol du ω-undecelynoyl phénylalanine, les ω-undecelynoyl dipeptides, l'arbutine, l'acide kojique, l'hydroquinone ; les composés montrant une action apaisante notamment le SEPICALM^{™} S, l'allantoïne et le bisabolol ; les agents anti-inflammatoires ; les composés montrant une action hydratante comme l'urée, les hydroxyurées, le glycérol, les polyglycérols, le glycérolglucoside, le diglycérolglucoside, les polyglycérylglucosides, le xylitylplucoside ; les extraits végétaux riches en polyphénols comme les extraits de raisin, les extraits de pin, les extraits de vin, les extraits d'olives; les composés montrant une action amincissante ou lipolytique comme la caféine ou ses dérivés, l'ADIPOSUM^{™}, l'ADIPOLESS^{™}, la fucoxanthine ; les protéines N-acylées ; les peptides N-acylés comme le MATRIXIL^{™} ; les acides aminés N-acylés ; les hydrolysâts partiels de protéines N-acylés ; les acides aminés ; les peptides ; les hydrolysâts totaux de protéines ; les extraits de soja, par exemple la Raffermine^{™} ; les extraits de blé par exemple la TENSINE^{™} ou la GLIADINE^{™} ; les extraits végétaux, tels que les extraits végétaux riches en tanins, les extraits végétaux riches en isoflavones ou les extraits végétaux riches en terpènes ; les extraits d'algues d'eau douce ou marines ; les extraits de plantes marines ; les extraits marins en général comme les coraux ; les cires essentielles ; les extraits bactériens ; les céramides ; les phospholipides ; les composés montrant une action antimicrobienne ou une action purifiante, comme le LIPACIDE^{™} C8G, le LIPACIDE^{™} UG, le SEPICONTROL^{™} A5 ; l'OCTOPIROX^{™} ou le SENSIVA^{™} SC50 ; les composés montrant une propriété énergisante ou stimulante comme le PHYSIOGENYL^{™}, le panthénol et ses dérivés comme le SEPICAP^{™} MP ; les actifs anti-âge comme le SEPILIFT^{™} DPHP, le LIPACIDE^{™} PVB, le SEPIVINOL^{™}, le SEPIVITAL^{™}, le MANOLIVA^{™}, le PHYTO-AGE^{™}, le TIMECODE^{™} ; le SURVICODE^{™} ; les actifs anti-photo vieillissement ; les actifs protecteurs de l'intégrité de la jonction dermoépidermique ; les actifs augmentant la synthèse des composants de la matrice extracellulaire comme le collagène, les élastines, les glycosaminoglycanes ; les actifs agissant favorablement sur la communication cellulaire chimique comme les cytokines ou physiques comme les intégrines ; les actifs créant une sensation de « chauffe » sur la peau comme les activateurs de la microcirculation cutanée (comme les dérivés de l'acide nicotinique) ou des produits créant une sensation de « fraîcheur » sur la peau (comme le menthol et des dérivés) ; les actifs améliorant la microcirculation cutanée, par exemple les veinotoniques ; les actifs drainants ; les actifs à visée décongestionnante comme les extraits de ginko biloba, de lierre, de marron d'inde, de bambou, de ruscus, de petit houx, de *centalla asiatica,* de fucus, de romarin, de saule ; les agents de bronzage ou de brunissement de la peau, par exemple la dihydroxyacétone (DHA), l'érythrulose, l'aldéhyde mésotartrique, le glutaraldéhyde, le glycéraldéhyde, l'alloxane, la ninhydrine, les extraits végétaux par exemple les extraits de bois rouges du genre Pterocarpus et du genre Baphia comme le Pteropcarpus santalinus, le Pterocarpus osun, le Pterocarpus soyauxii, le Pterocarpus erinaceus, le Pterocarpus indicus ou le Baphia nitida comme ceux décrits dans la demande de brevet Européen EP 0 971 683 ; les agents connus pour leur action de facilitation et/ou d'accélération du bronzage et/ou du brunissement de la peau humaine, et/ou pour leur action de coloration de la peau humaine, par exemple les caraténoïdes ( et plus particulièrement le beta carotène et le gamma carotène), le produit commercialisé sous le nom de marque « Carrot oil » (Nom INCI: Daucus Carota, helianthus annuus Sunflower oil) par la société Provital, qui contient des caroténoïdes, de la vitamine E et de la vitamine K; la tyrosine et/ou ses dérivés, connus pour leur effet sur l'accélération du bronzage de la peau humaine en association avec une exposition aux rayonnements ultra-violets, par exemple le produit commercialisé sous le nom de marque « SunTan Accelerator^{™} » par la société Provital qui contient de la tyrosine et des riboflavines (vitamine B), le complexe de tyrosine et de tyrosinase commercialisé sous le nom de marque « Zymo Tan Complex » par la société Zymo Line, le produit commercialisé sous le nom de marque MelanoBronze^{™} (nom INCI : Acetyl Tyrosine, Monk's pepper extract (Vitex Agnus-castus)) par la société Mibelle qui contient de l'acétyl tyrosine, produit commercialisé sous le nom de marque Unipertan VEG-24/242/2002 (nom INCI: butylene glycol and Acetyl Tyrosine and hydrolyzed vegetable protein and Adenosine triphosphate) par la société UNIPEX, le produit commercialisé sous le nom de marque « Try-Excell^{™} » (nom INCI: Oleoyl Tyrosine and Luffa Cylindrica (Seed) Oil and Oleic acid) par la société Sederma qui contient des extraits de pépins de courge (ou huile de Loofah), le produit commercialisé sous le nom de marque «Actibronze^{™} » (nom INCI: hydrolyzed wheat protein and acetyl tyrosine and copper gluconate) par la société Alban Muller, le produit commercialisé sous le nom de marque Tyrostan^{™} (nom INCI : potassium caproyl tyrosine) par la société Synerga, le produit commercialisé sous le nom de marque Tyrosinol (nom INCI: Sorbitan Isostearate, glyceryl oleate, caproyl Tyrosine) par la société Synerga, le produit commercialisé sous le nom de marque InstaBronze^{™} (nom INCI : Dihydroxyacetone and acetyl tyrosine and copper gluconate) commercialisé par la société Alban Muller, le produit commercialisé sous le nom de marque Tyrosilane (nom INCI : méthylsilanol and acétyl tyrosine) par la société Exymol ; les peptides connus pour leur effet d'activation de la mélanogénèse par exemple le produit commercialisé sous le nom de marque Bronzing SF Peptide powder (nom INCI: Dextran and Octapeptide-5) par la société Infinitec Activos, le produit commercialisé sous le nom de marque Melitane (nom INCI : Glycerin and Aqua and Dextran and Acetyl hexapeptide-1) comprenant l'acétyl hexapeptide-1 connu pour son action agoniste de l'alpha-MSH, le produit commercialisé sous le nom de marque Melatimes Solutions^{™} (nom INCI: Butylene glycol , Palmitoyl Tripeptide-40) par la société LIPOTEC, les sucres et les dérivés de sucres par exemple le produit commercialisé sous le nom de marque Tanositol^{™} (nom INCI: inositol) par la société Provital, le produit commercialisé sous le nom de marque Thalitan^{™} (ou Phycosaccharide^{™} AG) par la société CODIF international (nom INCI: Aqua and hydrolyzed algin (Laminaria Digitata) and magnesium sulfate and manganese sulfate) contenant un oligosaccharide d'origine marine (acide guluronique et acide mannuronique chélatés avec les ions magnésium et manganèse), le produit commercialisé sous le nom de marque Melactiva^{™} (nom INCI: Maltodextrin, Mucuna Pruriens Seed extract) par la société Alban Muller, les composés riches en flavonoïdes par exemple le produit commercialisé sous le nom de marque « Biotanning » (nom INCI: Hydrolyzed citrus Aurantium dulcis fruit extract) par la société Silab et connu pour être riche en flavonoides de citron (de type hespéridines) ; les agents destinés au traitement des cheveux et/ou des poils, par exemple des agents protecteurs des mélanocytes du follicule pileux, destinés à protéger lesdits mélanocytes contre les agents cytotoxiques responsables de la sénescence et/ou de l'apoptose desdits mélanocytes, tels que les agents mimétiques de l'activité de la DOPAchrome tautomérase choisis parmi ceux décrits dans la demande de brevet européen publiée sous le numéro EP1515688 A2, les molécules synthétiques mimétiques de la SOD par exemples les complexes de manganèse, des composés antioxydants par exemple les dérivés de cyclodextrine, des composés silicés dérivés d'acide ascorbique, de la pyrrolidone carboxylate de lysine ou d'arginine, , des associations de mono- et diester d'acide cinnamique et de vitamine C, et plus généralement ceux cités dans la demande de brevet européen publiée sous le numéro EP 1515 688 A2.

Comme exemples d'agents antioxydants que l'on peut associer à la composition (CA) dans les formulations cosmétiques à usage topique , on peut citer l'EDTA et ses sels, l'acide citrique, l'acide tartarique, l'acide oxalique, le BHA (butylhydroxyanisol), le BHT (butylhydroxytoluène), les dérivés de tocophérol tels que l'acétate de tocophérol, des mélanges de composés antioxydants tels que la DISSOLVINE 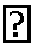 GL 47S commercialisé par la société Akzo Nobel sous le nom INCI: Tetrasodium Glutamate Diacetate. Comme exemples de filtres solaires que l'on peut associer à la composition (CA) dans les formulations cosmétiques à usage topique , on peut citer tous ceux figurant dans la directive cosmétique 76/768/CEE modifiée annexe VII. Parmi les filtres organiques solaires que l'on peut associer au composé de formule (I) ou à la composition (C1) dans les formulations cosmétiques à usage topique selon l'invention, on peut citer la famille des dérivés de l'acide benzoïque comme les acides para-aminobenzoïques (PABA), notamment les esters de monoglycérol de PABA, les esters éthyliques de N,N-propoxy PABA, les esters éthyliques de N,N-diéthoxy PABA, les esters éthyliques de N,N-diméthyl PABA, les esters méthyliques de N,N-diméthyl PABA, les esters butyliques de N,N-diméthyl PABA; la famille des dérivés de l'acide anthranilique comme l'homomenthyl-N-acétyl anthranilate ; la famille des dérivés de l'acide salicylique comme le salicylate d'amyle, le salicylate d'homomenthyle, le salicylate d'éthylhexyle, le salicylate de phényle, le salicylate de benzyle, le salicylate de p-isopropanolphényle ; la famille des dérivés de l'acide cinnamique comme le cinnamate d'éthylhexyle, le cinnamate d'éthyl-4-isopropyle, le cinnamate de méthyl-2,5-diisopropyle, le cinnamate de p-méthoxypropyle, le cinnamate de p-méthoxyisopropyle, le cinnamate de p-méthoxyisoamyle, le cinnamate de p-méthoxyoctyle (le cinnamate de p-méthoxy 2-éthylhexyle), le cinnamate de p-méthoxy 2-éthoxyéthyle, le cinnamate de p-méthoxycyclohexyle, le cinnamate d'éthyl-α-cyano-β-phényle, le cinnamate de 2-éthylhexyl- 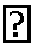 α-cyano-β-phényle, le cinnamate de diparaméthoxy mono-2-éthylhexanoyl de glycéryle ; la famille des dérivés de la benzophénone comme la 2,4-dihydroxybenzophénone, la 2,2'-dihydroxy-4-méthoxybenzophénone, la 2,2',4,4'-tétrahydroxybenzophénone, la 2-hydroxy-4-méthoxybenzophénone, la 2-hydroxy-4-méthoxy-4'-méthylbenzophénone, la 2-hydroxy-4-méthoxybenzophénone-5-sulfonate, la 4-phénylbenzophénone, le 2-éthylhexyl-4'-phénylbenzophénone-2-carboxylate, la 2-hydroxy-4-n-octyloxybenzophénone, la 4-hydroxy-3-carboxybenzophénone ; le 3-(4'-méthylbenzylidène)-d,l-camphre, le 3-(benzylidène)-d,l-camphre, le benzalkonium méthosulfate camphre ; l'acide urocanique, l'urocanate d'éthyle ; la famille des dérivés de l'acide sulfonique comme l'acide sulfonique 2-phénylbenzimidazole-5 et ses sels; la famille des dérivés de la triazine comme l'hydroxyphényl triazine, l'éthylhexyloxyhydroxyphényl-4-méthoxyphényltriazine, le 2,4,6-trianillino-(p-carbo-2'-éthylhexyl-1'-oxy)-1,3,5-triazine, le 4,4-((6-(((1,1-diméthyléthyl) amino)carbonyl)phenyl)amino)-1,3,5-triazine-2,4-diyl diimino) bis-(2-éthylhexyl) ester de l'acide benzoïque, le 2-phényl-5-méthylbenzoxazole, le 2,2'-hydroxy-5-méthylphénylbenzotriazole, le 2-(2'-hydroxy-5'-t-octylphényl)benzotriazole, le 2-(2'-hydroxy-5'-méthyphényl)benzotriazole; la dibenzazine; le dianisoylméthane, le 4-méthoxy-4"-t-butylbenzoylméthane ; la 5-(3,3-diméthyl-2-norbornylidène)-3-pentan-2-one ; la famille des dérivés du diphénylacrylate comme le 2-éthylhexyl-2-cyano-3,3-diphényl-2-propènoate, l'éthyl-2-cyano-3,3-diphényl-2-propènoate ; la famille des polysiloxanes comme le malonate de benzylidène siloxane.

Parmi les filtres inorganiques solaires, également appelés "écrans minéraux", que l'on peut associer à la composition (CA) dans les formulations cosmétiques à usage topique , on peut citer les oxydes de titane, les oxydes de zinc, l'oxyde de cérium, l'oxyde de zirconium, les oxydes de fer jaune, rouge ou noir, les oxydes de chrome. Ces écrans minéraux peuvent être micronisés ou non, avoir subi ou non des traitements de surface et être éventuellement présentés sous formes de pré-dispersions aqueuses ou huileuses. L'exposé expérimental suivant illustre l'invention sans toutefois la limiter.

### A-1) - Choix du procédé de sélection

### A-1-1) Choix du type d'induction de la sénescence

Quatre différents types d'induction de la sénescence ont été étudiés :
- L'exposition de fibroblastes « jeunes » pris au passage R6 aux rayonnements ultraviolet A de façon aiguë ; plusieurs doses de rayonnement ont été testées : 5, 10, 15 ou 20J/cm², pour une exposition par jour ;
- L'exposition de fibroblastes « jeunes » pris au passage R6 aux rayonnements ultraviolet A de façon chronique ; plusieurs doses de rayonnement ont été testées : 3x 5 J/cm² ou 6x 2,5 J/cm², pour deux expositions par jour ;
- L'utilisation de fibroblastes « vieillis artificiellement » par réplications successives jusqu'au passage R18 ;
- L'utilisation d'un pool de fibroblastes issus de donneurs âgés (moyenne de 58 ans)

Il est à noter que les fibroblastes jeunes (passage R6) et sénescents (passage R20) sont issus de mêmes donneurs et du même lot de cellules. Les fibroblastes pris au passage R20 ont été obtenus après des réplications successives des cellules R6.

Pour évaluer le type d'induction le plus adapté, les cellules fibroblastes, que ce soit celles prises au passage R6 ou au passage R20, ont été ensemencées en plaques 48 puits à 15000 ou 10000 cellules/puits, dans un milieu de culture classique pour fibroblastes, à 37°C dans une atmosphère humide contenant 5% de CO₂.

Chaque condition a été réalisée en *quadruplicate.*

Le vieillissement contagieux ou effet bystander a ensuite été évalué par mesure de la quantité d'IL-6 extracellulaire et de l'activité de la β-galactosidase :
- pour l'exposition aux UVA en chronique ou en aigüe
- à 96h et 144h pour l'utilisation du pool de fibroblastes issus de donneurs âgés.
- à 24h et 48h pour la sénescence induite par réplication.

Les résultats obtenus ont permis ensuite de calculer des taux d'évolution de la quantité d'IL-6 extracellulaire et de l'activité de la β-galactosidase, et de recueillir des observations qualitatives ; tous ces résultats étant compris dans le tableau 1 ci-dessous.

**Tableau 1**

| Type d'induction | Expositions aiguës aux UVA | Expositions chroniques aux UVA | sénescence réplicative | contact avec fibroblastes âgés |
|---|---|---|---|---|
| Paramètres de l'induction | 5-10-15-20 J/cm² | 3x5 J/cm² ou 6x2,5 J/cm² | MCR20 | Pool jeune vs pool vieux |
| Marqueur biologique IL-6 | Taux d'augmentation IL-6 >100% dès 4 heures | Non mesurable | Taux évolution après 48 heures : 59% | Taux évolution après 96 heures : 230% |
| Marqueur biologique β-galactosidase | Marquage trop élevé car confluence trop importante | Très peu de marquage pour Témoin négatif et UVA | Taux évolution après 48 heures : 378% | Taux évolution après 48 heures : diminution observée |

Seule la réplication des fibroblastes jusqu'au passage R20 a permis d'observer une augmentation de la sécrétion de l'IL-6 et une augmentation du marquage de la β-galactosidase caractéristique d'un phénotype de vieillissement contagieux ou effet bystander.

L'essai ci-dessus a donc permis de sélectionner la réplication successive comme type d'induction de la sénescence pour le modèle mimant le vieillissement contagieux.

### A-1-2) Choix du modèle le plus adapté pour mimer le vieillissement contagieux

Différentes méthodes ont été utilisées pour sélectionner laquelle était la plus adaptée pour observer "l'effet bystander" sur les cellules fibroblastes prises aux passages R6 et R20 :
- Co-culture indirecte, soit avec insertion, des cellules fibroblastes jeunes R6 et des cellules fibroblastes sénescentes R20 dans du milieu de culture classique pour fibroblastes
- Co-culture directe de ces cellules dans un même puits avec marquage des cellules sénescentes prises au passage R20 pour les différencier
- Utilisation de milieux conditionnés MCR20 de cellules sénescentes, fibroblastes R20, mis en contact avec des fibroblastes "jeunes" prises passage R6.

### Description des protocoles utilisés pour déterminer le modèle le plus adapté à l'évaluation du vieillissement contagieux :

### Modèle de co-culture indirecte utilisant des inserts :

Des fibroblastes humains normaux pris en passage R5 et R19 ont été amplifiés respectivement jusqu'aux passages respectifs R6 et R20 puis ensemencés en plaques 24 puits à 30000 cellules/puits pour les R6 et en insert à 15000 cellules/puits pour les R20.

Après une amplification de 72 heures en inserts et en plaques 24 puits, les inserts, contenant les cellules R20, ont été déposés dans les plaques 24 puits contenant les cellules R6 et mis en contact pendant 24h et 4 jours.

Après ces incubations de 24h et 4 jours, les marqueurs de sénescence suivants ont été évalués :
- l'activité de la β-galactosidase par coloration
- la quantité d'interleukine-6 par dosage ELISA

### Modèle de co-culture directe :

Des fibroblastes humains normaux en pris en passage R5 et R19 ont été amplifiés respectivement jusqu'aux passages respectifs R6 et R20 puis ensemencés en boîtes de petri à 0,3M/boite de pétri.

Après 5 jours d'amplification en boîtes de pétri, les tapis cellulaires des cellules au passage R20 ont été marqués avec de la fluorescence (marqueur Alexa Fluor^{®} 488 NHS Ester à 5mM). Ils ont ensuite été ensemencés dans des plaques 48 puits avec des cellules R6 non sénescents et non marqués au ratio 1:1 (soit 7500 cellules/puits pour les 2 passages). Les cellules ainsi ensemencées ont ensuite été incubées 48h.

Après cette incubation de 48h, les marqueurs de sénescence suivants ont été évalués :
- l'activité de la β-galactosidase par coloration
- la quantité d'interleukine-6 par dosage ELISA

### Modèle utilisant des milieux conditionnés :

Des fibroblastes humains normaux pris au passage R19 ont été amplifiés jusqu'au passage R20 puis ensemencés en boîtes de pétri à 0,3M/boite de pétri.

Après une amplification de 4 jours en boîtes de pétri, le milieu de culture de toutes les boîtes a été changé puis récupéré après 48h d'incubation. Ce milieu récupéré correspond au milieu conditionné de cellules sénescentes contenant les médiateurs du SMS (MCR20).

En parallèle, des fibroblastes "jeunes" en R6 ont été ensemencés en plaques 48 puits à 10000 et 15000 cellules/puits. Après 72h d'incubation, le milieu conditionné MCR20 a été déposé sur ces cellules. Les cellules ainsi traitées avec le milieu conditionné de cellules sénescentes ont été incubées 24h et 48h.

Les marqueurs de sénescence suivants ont été ensuite évalués :
- l'activité de la β-galactosidase par coloration à 48h
- la quantité d'interleukine-6 par dosage ELISA à 24h

### Conclusions

Des résultats concluants et fiables ont été mis en évidence pour le seul modèle de vieillissement contagieux, utilisant les milieux conditionnés : augmentation de l'interleukine-6, et modification de leur activité métabolique (augmentation de l'activité de la β-galactosidase). Ces modifications sont caractéristiques d'une sénescence des cellules "jeunes" traitées avec le milieu conditionné de cellules vieillies.

### A-2) - Définition du procédé optimal.

Le procédé particulier comprend donc les étapes suivantes :
- Etape i) de mise en culture de de mise en culture de fibroblastes du derme humain, pris au passage R19 dans des flasques T25,
- Etape ii) d'amplification des cellules obtenues à l'issue de l'étape i) pour obtenir un milieu conditionné de cellules sénescentes ou «MC R20 ».

L'amplification des cellules obtenues à l'issue de l'étape i) est réalisée par ensemencement desdites cellules en boîtes de pétri à 0,3M/boite de pétri. Après une amplification de 4 jours en boîtes de pétri, le milieu de culture de toutes les boîtes a été changé puis récupéré après 48h d'incubation. Ce milieu récupéré correspond au milieu conditionné de cellules sénescentes contenant les médiateurs du SMS (MCR20).
- Etape iii) de mise en culture de fibroblastes du derme humain, pris au passage R5, dans des flasques T25,
- une étape iv) d'amplification des cellules obtenues à l'issue de l'étape iii) pour obtenir pour obtenir des cellules « jeunes » ou « R6 » en phase d'amplification,

### Les étapes iii) et iv) sont menées en parallèle des étapes i) et ii).

L'amplification des cellules obtenues à l'issue de l'étape iii) est réalisée par ensemencement desdites cellules en plaques 48 puits à 10000 et 15000 cellules/puits.
- une étape v) de mise en contact d'une substance chimique (S) ou d'une composition chimique (C) avec le milieu cellulaire obtenu à l'étape iv),
- une étape vi) de mise en contact du milieu conditionné MCR20e obtenu à l'étape ii) avec les cellules "jeunes" ou "R6" en phase d'amplification obtenues à l'étape iii),

Dans cette étape d), le milieu conditionné MCR20 obtenu à l'étape ii) est mis en contact avec les cellules "jeunes" ou "R6" obtenues à l'étape v) pendant 48 heures.
- une étape vii) de mesure de la quantité de l'Interleukine IL-6 extracellulaire présente dans le surnageant de culture et une mesure de l'activité de la β-galactosidase présente dans les cellules issues de l'étape vi).
- une étape viii) de comparaison des niveaux d'expression de l'IL-6 extracellulaire et de la β-galactosidase

De façon, plus précise, on calcule dans le cadre de cette étape viii) :
Le rapport R1= [(N10 - N1i) x 100] / [(N10 - N1)] pour l'interleukine IL-6 extracellulaire avec:
   - N1 correspondant à la quantité d'IL-6 mesurée dans le milieu de culture obtenue à l'issue du procédé tel que décrit ci-dessus, sans mettre en œuvre ni l'étape v) et ni l'étape vi) du procédé (cellules au passage R6, non traitées et non associées aux cellules conditionnées du passage R20),
   - N10 correspondant à la quantité d'IL-6 mesurée dans le milieu de culture obtenue à l'issue du procédé tel que décrit ci-dessus, sans mettre en œuvre l'étape v) du procédé (cellules au passage R6, non traitées, et associées aux cellules conditionnées du passage R20),
   - N1i correspondant à la quantité d'IL-6 mesurée dans le milieu de culture obtenue à l'issue du procédé tel que décrit ci-dessus, lorsqu'une substance ou une composition (i) est mise en œuvre dans l'étape v) du procédé (cellules traitées associées aux cellules conditionnées du passage R20).

Le rapport R2 = [(N20 - N2i) x 100] / [(N20 - N2)] pour l'interleukine IL-6 extracellulaire avec:
- N2 correspondant à la quantité de la β-galactosidase mesurée dans le milieu de culture obtenue à l'issue du procédé tel que décrit ci-dessus, sans mettre en œuvre ni l'étape v) et ni l'étape vi) du procédé (cellules au passage R6, non traitées et non associées aux cellules conditionnées du passage R20),
- N20 correspondant à la quantité de la β-galactosidase mesurée dans le milieu de culture obtenue à l'issue du procédé tel que décrit ci-dessus, sans mettre en œuvre l'étape v) du procédé (cellules au passage R6, non traitées, et associées aux cellules conditionnées du passage R20),
- N2i correspondant à la quantité de la β-galactosidase mesurée dans le milieu de culture obtenue à l'issue du procédé tel que décrit ci-dessus, lorsqu'une substance ou une composition (i) est mise en œuvre dans l'étape v) du procédé (cellules traitées et associées aux cellules conditionnées du passage R20).

### B]- Evaluation de substances et de compositions selon le procédé objet de la présente invention.

La composition (CA1) a été préparée par extraction à l'aide d'une poudre de l'algue rouge *Asparagopsis armata* préalablement séchée et tamisée, par un mélange 1,3-propanediol/eau (40/60 v/v) de sorte à obtenir un extrait *d'Asparagopsis armata* à 1,2 % dans ledit solvant.

La composition (CA2) a été préparée par macération dans l'éthanol à une température de 20°C pendant une durée de deux heures. L'extrait ainsi obtenu est évaporé et repris au méthanol, ce qui fait précipiter les minéraux présents, qui sont éliminés par filtration. Le filtrat est ensuite évaporé puis purifié par HPLC semi-préparative (Colonne C18 phenomenex^{™}) selon un gradient de solvant H₂0:CH₃CN, et la fraction recueillie a été conservée, puis analysée pour déterminer une quantité de mycosporine-acide aminé à hauteur de 50 mg/L en équivalent palythine.

Les résultats obtenus pour chacune des compositions, suite à la mise en œuvre du procédé précédemment décrit sont consignés dans le Tableau 2 ci-dessous :
Les compositions (CA1) et (CA2) ont été ensuite diluées dans le 1,3-propanediol à hauteur de 0,1% massique.

**Tableau 2**

| Produits testés | IL-6 extracellulaire (pg/µg de protéines) | β-galactosidase (nombre de cellules positives "bleues" / nombre total de cellules) |
|---|---|---|
| Cellules « jeunes »passage R6 non traitées, non mises en contact avec le milieu conditionné MCR20 produit par les cellules « sénescentes » passage R20 | N1 = 8,96 ± 0,37 | N2 = 3,04 ± 0,78 |
| Cellules « jeunes » passage R6 non traitées, après mise en contact avec le milieu conditionné MCR20 produit par les cellules « sénescentes » passage R20 | N1_{0 =} 41,38 ± 9,11 | N2₀= 9,66 ± 1,65 |
| Cellules « jeunes » passage R6 après traitement Vitamine C et mise en contact avec le milieu conditionné MCR20 produit par les cellules « sénescentes » passage R20 | N1i = 27,31± 3,87 | n.r |
| | R1 = 43,4 | |
| Cellules « jeunes » passage R6 après traitement Vitamine E(^{∗}) et mise en contact avec le milieu conditionné MCR20 produit par les cellules « sénescentes » passage R20 | n.r | N2i = 2,99 ± 0,45 |
| | | R2 = 100,75 |
| Cellules « jeunes » passage R6 après traitement Composition (CA1) et mise en contact avec le milieu conditionné MCR20 produit par les cellules « sénescentes » passage R20 | N1i = 29,81 ± 3,30 | N2i = 3,20± 0,61 |
| | R1 =35,7 | R2 = 97,6 |
| Cellules « jeunes » passage R6 après traitement Composition (CA2) et mise en contact avec le milieu conditionné MCR20 produit par les cellules « sénescentes » passage R20 | N1i = 29,45 ± 0,25 | N2i = 4,41 ± 1,54 |
| | R1 = 36,8 | R2 = 79,3 |
| Cellules « jeunes » passage R6 après traitement 1,3 Propanediol et mise en contact avec le milieu conditionné MCR20 produit par les cellules « sénescentes » passage R20 | N1i = 37,05 ± 2,57 | N2i = 13,40 ± 2,69 |
| | R1 = 13,35 | R2 = - 56,5 |

| | | |
|---|---|---|
| (^{∗}) : la Vitamine E est de l'acétate de D-α tocophérol | | |

### Analyse des résultats

Les rapports R1 sont supérieurs à 30 à la fois pour la vitamine C utilisé comme composé de référence, et pour les compositions (CA1) et (CA2).

Les rapports R2 sont supérieurs à 70 à la fois pour la vitamine E utilisé comme composé de référence, et pour les compositions (CA1) et (CA2).

Ces compositions (CA1) et (CA2) peuvent être sélectionnées pour protéger contre le vieillissement contagieux ou effet bystander.

### Bibliographie

[1] : Campisi, J and d'Adda di Fagagna F, "Cellular senescence : when bad things happen to good cells." Nat.Rev., Mol Cell Biol, 2007, 8, 729-740)
[2] : Coppe JP et al, "Senescence-associated secretory phenotypes reveal cellnonautonomous functions of oncogenic RAS and the p53 tumor suppressor", PloS Biol, 2008, 6, 2853-2868
[3] : Passos JF et al, A feedback between p21 and reactive oxygen production is necessary for cell senescence, Mol. Syst. Biol, 2010, 6, 347)
[4] : Nelson G, Wordsworth J, Wang C, Jurk D, Lawless C, Martin-Ruiz C. and Von Zglinicki T. : "A senescent cell bystander effect: senescence-induced Senescence". Aging Cell, 2012, 11, 345-349.
[5] : Redmond R.W, Rajadurai A, Udayakumar D, Sviderskaya E.V, Tsao H. "Melanocytes are selectively vulnerable to UVA-mediated Bystander Oxidative Signaling." J. Invest Dermatol. 2014, 134, pp. 1083-1090.
[6] Demande de brevet FR 2 971 792 A1

## Revendications

1. Procédé pour évaluer la capacité d'une substance chimique (S) ou d'une composition chimique (C) à prévenir ou à ralentir l'apparition des signes du vieillissement de la peau humaine ou des lèvres ou bien d'éliminer lesdits signes ; ledit procédé comprenant :
- une étape a) de mise en contact de la substance chimique ou de la composition chimique avec des cellules de fibroblastes du derme humain « jeunes » prises dans un milieu de culture au passage R6,
- un étape b) de mise en contact de cellules sénescentes de fibroblastes du derme humain avec les cellules de fibroblastes « jeunes » issues de l'étape a), et
- une étape c) de mesure de l'entrée en sénescence des cellules de fibroblastes « jeunes » et de comparaison avec une valeur de référence.

2. Procédé selon la revendication 1, **caractérisé en ce que** les cellules sénescentes sont des cellules de fibroblastes du derme humain pris dans un milieu de culture au passage R20.

3. Procédé selon la revendication 1, **caractérisé en ce que** les cellules sénescentes sont des cellules de fibroblastes du derme humain qui ont été pris dans un milieu de culture au passage R19 avant de subir une amplification.

4. Procédé selon l'une des revendications 1 à 3, **caractérisé en ce que** les cellules de fibroblastes du derme humain « jeunes » prises dans un milieu de culture au passage R6 sont issues d'une amplification de cellules de fibroblastes du derme humain « jeunes » prises dans un milieu de culture au passage R5.

5. Procédé selon l'une des revendications 1 à 4, **caractérisé en ce que** l'étape c) de mesure de l'entrée en sénescence des cellules de fibroblastes « jeunes » comprend l'utilisation d'au moins deux marqueurs biologiques :
- au moins un marqueur biologique (M1) du SASP/SMS, et
- au moins un marqueur biologique (M2) de la sénescence.

6. Procédé selon la revendication 5, **caractérisé en ce que** le marqueur biologique (M1) du SASP/SMS est choisi parmi les éléments du groupe constitué par les cytokines proinflammatoires, et plus particulièrement l'interleukine-1 (IL-1) extracellulaire, l'interleukine-6 (IL-6) extracellulaire, l'interleukine-8 (IL-8) extracellulaire, des facteurs de croissance, les enzymes de dégradation de la matrice telles que la MMP-1 et la MMP-3, des espèces réactives de l'oxygène ou ROS.

7. Procédé selon l'une des revendications 5 ou 6, **caractérisé en ce que** le marqueur biologique (M2) de la sénescence est choisi parmi les éléments du groupe constitué par la β-galactosidase, les dommages à l'ADN persistants, et une résistance à l'apoptose.

8. Procédé selon l'une des revendications 5 à 7, **caractérisé en ce que** le marqueur biologique (M1) est l'Interleukine-6 et le marqueur biologique (M2) est l'enzyme β-galactosidase.

9. Procédé selon l'une des revendications 5 à 8, **caractérisé en ce qu'**à l'étape c) on compare les niveaux d'expressions des deux marqueurs biologiques (M1) et (M2) avec au moins un niveau de d'expression de référence pour chacun de ces deux marqueurs biologiques.

10. Procédé selon l'une des revendications 1 à 9, **caractérisé en ce que** les signes du vieillissement de la peau humaine ou des lèvres sont choisis parmi
- l'apparition des rides, des ridules ou d'une altération du microrelief ; et/ou
- l'apparition du manque d'élasticité et/ou de tonus ; et/ou
- l'apparition du manque de densité et/ou de fermeté.

## Patentansprüche

1. Verfahren zur Beurteilung der Fähigkeit einer Chemikalie (S) oder einer chemischen Zusammensetzung (C), die Symptome der Alterung der menschlichen Haut oder der Lippen zu verhindern oder zu verlangsamen oder die Symptome sogar zu beseitigen; wobei das Verfahren Folgendes umfasst:
- einen Schritt a) des In-Kontakt-Bringens der Chemikalie oder der chemischen Zusammensetzung mit "jungen" Fibroblastenzellen der menschlichen Dermis, die in der Passage R6 aus einem Kulturmedium entnommen werden,
- einen Schritt b) des In-Kontakt-Bringens von alternden Fibroblastenzellen der menschlichen Dermis mit den "jungen" Fibroblastenzellen aus Schritt a) und
- einen Schritt c) des Messens des Eintritts der "jungen" Fibroblastenzellen in die Seneszenz und des Vergleichs mit einem Referenzwert.

2. Verfahren nach Anspruch 1, **dadurch gekennzeichnet, dass** es sich bei den alternden Zellen um Fibroblastenzellen von menschlicher Dermis handelt, die in der Passage R20 aus einem Kulturmedium entnommen werden.

3. Verfahren nach Anspruch 1, **dadurch gekennzeichnet, dass** es sich bei den alternden Zellen um Fibroblastenzellen von menschlicher Dermis handelt, die in der Passage R19 aus einem Kulturmedium entnommen wurden, bevor sie einer Amplifikation unterzogen werden.

4. Verfahren nach einem der Ansprüche 1 bis 3, **dadurch gekennzeichnet, dass** die "jungen" Fibroblastenzellen der menschlichen Dermis, die in Passage R6 aus einem Kulturmedium entnommen werden, aus einer Amplifikation von "jungen" Fibroblastenzellen der menschlichen Dermis stammen, die in Passage R5 aus einem Kulturmedium entnommen wurden.

5. Verfahren nach einem der Ansprüche 1 bis 4, **dadurch gekennzeichnet, dass** Schritt c) des Messens des Eintritts der "jungen" Fibroblastenzellen in die Seneszenz die Verwendung von mindestens zwei biologischen Markern umfasst:
- mindestens eines biologischen Markers (M1) von SASP/SMS und
- mindestens eines biologischen Markers (M2) der Seneszenz.

6. Verfahren nach Anspruch 5, **dadurch gekennzeichnet, dass** der biologische Marker (M1) von SASP/SMS ausgewählt ist aus den Elementen der Gruppe, die aus den proinflammatorischen Zytokinen und insbesondere aus extrazellulärem Interleukin-1 (IL-1), extrazellulärem Interleukin-6 (IL-6), extrazellulärem Interleukin-8 (IL-8), Wachstumsfaktoren, Matrixabbauenzymen wie MMP-1 und MMP-3, reaktiven Sauerstoffspezies oder ROS besteht.

7. Verfahren nach einem der Ansprüche 5 oder 6, **dadurch gekennzeichnet, dass** der biologische Marker (M2) der Seneszenz ausgewählt ist aus den Elementen der Gruppe, die aus β-Galactosidase, persistierenden DNA-Schädigungen und einer Apoptoseresistenz besteht.

8. Verfahren nach einem der Ansprüche 5 bis 7, **dadurch gekennzeichnet, dass** es sich beim biologischen Marker (M1) um Interleukin-6 handelt und beim biologischen Marker (M2) um das Enzym β-Galactosidase handelt.

9. Verfahren nach einem der Ansprüche 5 bis 8, **dadurch gekennzeichnet, dass** Schritt c), in dem die Expressionsgrade der beiden biologischen Marker (M1) und (M2) mit mindestens einem Referenzexpressionsgrad für jeden dieser beiden biologischen Marker verglichen werden.

10. Verfahren nach einem der Ansprüche 1 bis 9, **dadurch gekennzeichnet, dass** die Symptome der Alterung der menschlichen Haut oder der Lippen ausgewählt sind aus
- dem Auftreten von Falten, Fältchen oder einer Veränderung des Mikroreliefs und/oder
- dem Auftreten eines Mangels an Elastizität und/oder an Tonus und/oder
- dem Auftreten eines Mangels an Dichte und/oder an Festigkeit.

## Claims

1. Process for evaluating the capacity of a chemical substance (S) or of a chemical composition (C) for preventing or slowing down the appearance of the signs of aging of human skin or the lips or for eliminating said signs; said process comprising:
- a step a) of placing the chemical substance or the chemical composition in contact with "young" human dermal fibroblast cells taken from a culture medium at the R6 passage,
- a step b) of placing human senescent dermal fibroblast cells in contact with "young" fibroblast cells obtained from step a), and
- a step c) of measuring the entry into senescence of the "young" fibroblast cells and of comparison with a reference value.

2. Process according to Claim 1, **characterized in that** the senescent cells are human dermal fibroblast cells taken from a culture medium at the R20 passage.

3. Process according to Claim 1, **characterized in that** the senescent cells are human dermal fibroblast cells which have been taken from a culture medium at the R19 passage before undergoing amplification.

4. Process according to one of Claims 1 to 3, **characterized in that** the "young" human dermal fibroblast cells taken from a culture medium at the R6 passage are obtained from an amplification of "young" human dermal fibroblast cells taken from a culture medium at the R5 passage.

5. Process according to one of Claims 1 to 4, **characterized in that** step c) of measuring the entry into senescence of the "young" fibroblast cells comprises the use of at least two biological markers:
- at least one biological marker (M1) for SASP/SMS, and
- at least one biological marker (M2) for senescence.

6. Process according to Claim 5, **characterized in that** the biological marker (M1) for SASP/SMS is chosen from the elements of the group consisting of proinflammatory cytokines, and more particularly extracellular interleukin-1 (IL-1), extracellular interleukin-6 (IL-6), extracellular interleukin-8 (IL-8), growth factors, matrix degradation enzymes such as MMP-1 and MMP-3, and reactive oxygen species or ROS.

7. Process according to either of Claims 5 and 6, **characterized in that** the biological marker (M2) for senescence is chosen from the elements of the group consisting of β-galactosidase, persistent DNA damage, and apoptosis resistance.

8. Process according to one of Claims 5 to 7, **characterized in that** the biological marker (M1) is interleukin-6 and the biological marker (M2) is the β-galactosidase enzyme.

9. Process according to one of Claims 5 to 8, **characterized in that**, in step c), the levels of expression of the two biological markers (M1) and (M2) are compared with at least one reference level of expression for each of these two biological markers.

10. Process according to one of Claims 1 to 9, **characterized in that** the signs of aging of human skin or the lips are chosen from
- the appearance of wrinkles, fine lines or impairment of the microrelief; and/or
- the appearance of lack of elasticity and/or tonicity; and/or
- the appearance of lack of density and/or firmness.
